# EUROPEAN PATENT SPECIFICATION

(11) **EP 2 258 705 B1**
(45) Date of publication and mention of the grant of the patent: **18.06.2014**
(21) Application number: 09726706.6
(22) Date of filing: 02.04.2009
(51) Int. Cl.: C07D 513/04, A61K 31/496, A61P 31/00

(54) **PHARMACEUTICALLY ACCEPTABLE SALTS OF ANTI-INFECTIVE QUINOLONE COMPOUND**
PHARMAZEUTISCH AKZEPTABLE SALZE AUS EINER ANTIINFEKTIÖSEN CHINOLINVERINDUNG
SELS PHARMACEUTIQUEMENT ACCEPTABLES DE LA QUINOLONE, UN ANTI-INFECTIEUX

(30) Priority: 03.04.2008 CN 200810027211
(43) Date of publication of application: 08.12.2010
(73) Proprietor: Guangzhou Baiyunshan Pharmaceutical Co. Ltd. Guangzhou Baiyunshan Pharmaceutica Factory, Bauyun Section Guangzhou Guangdong 510515 (CN); Guangzhou Pharmaceutical Industry Academe, Guangdong 510240 (CN)
(72) Inventor: CHEN, Mao, Guangzhou Guangdong 510515 (CN); ZHU, Shaoxuan, Guangzhou Guangdong 510240 (CN); ZHENG, Lizhen, Guangzhou Guangdong 510515 (CN); LIU, Xuebin, Guangzhou Guangdong 510240 (CN); WANG, Yuping, Guangzhou Guangdong 510240 (CN); XU, Shuwen, Guangzhou Guangdong 510515 (CN)
(74) Representative: Von Kreisler Selting Werner - Partnerschaft von Patentanwälten und Rechtsanwälten mbB
(86) International application number: PCT/CN2009/071136
(87) International publication number: WO 2009/121303

(56) References cited:
- WO-A1-2007/082472
- WO-A1-2007/082472
- JP-A- 3 218 383
- JP-A- 3 218 383
- SEGAWA, J. ET AL.: "Studies on pyridonecarboxylic acids. IV Synthesis and antibacterial activity evaluation of S-(-)- and R-(+)-6-fluoro-l -methyl-4-oxo-7-(l - piperazinyl)-4H-[1,3]thiazeto[3,2-a]quinol ine-3-carboxylic acids", CHEMICAL & PHARMACEUTICAL BULLETIN, vol. 43, no. 7, 1995, pages 1238-1240, XP009144696,
- SEGAWA, J. ET AL.: 'Studies on pyridonecarboxylic acids. IV Synthesis and antibacterial activity evaluation of S-(-)- and R-(+)-6-fluoro-l -methyl-4-oxo-7-(l - piperazinyl)-4H-[1,3]thiazeto[3,2-a]quinoli ne-3-carboxylic acids' CHEMICAL & PHARMACEUTICAL BULLETIN vol. 43, no. 7, 1995, ISSN 0009-2363 pages 1238 - 1240

## Description

### FIELD OF THE INVENTION

This application is based on Chinese Patent Application No. 200810027211.9; the disclosure of the Chinese Patent Application is incorporated herein by reference.

This invention relates to a fluorine-containing, optically active quinolone compound for antibacterial drugs. More particularly, the invention relates to a pharmaceutically acceptable salt formed by (S)-6-fluoro-1-methyl-4-oxo-7-(1-piperazinyl)-1H,4*H*-[1,3]thiazeto[3,2-a]quinoline-3-carboxylic acid and an organic or inorganic acid.

### BACKGROUND OF THE INVENTION

Infectious diseases are the most common diseases involving virtually all clinical specialties. It is also one of the most common causes of death of patients. According to the report of the World Health Organization in 2000, the number of deaths caused by infectious diseases was 33.3% of the total number of deaths.

In the world market, the quinolone type drugs account for about 18% market share of the anti-infection drugs with an average annual growth rate of 7%. It continues to grow rapidly. Its total sales are second only to 13-lactam drugs. Fluoroquinolones antibacterial drugs are also developing rapidly in recent years because of their relatively broader antibacterial spectrum and antibacterial activity. Both oral and injectable formulations of fluoroquinolones antibacterial drugs are commonly used in clinical applications in China. The antibacterial mechanism of fluoroquinolone is targeting the DNA of the bacteria, blocking the bacterial DNA topoisomerase to form super-helical DNA, leading to irreversible damage to chromosomes, and preventing bacterial cell from division and breeding.

6-fluoro-1-methyl-4-oxo-7-(1-piperazinyl)-1H,4H-[1,3]thiazeto[3,2-a]quinoline-3-carboxylic acid is commonly called ulifloxacin internationally and "You Li Sha Xing" in Chinese, code NM394. Chinese invention patent CN101003540A disclosed the use of 6-fluoro-1-methyl -4-oxo-7-(1-piperazinyl)-1H,4*H*-[1,3]thiazeto[3,2-a]quinoline-3-carboxylic acid mesylate in the preparation of anti-infection drugs. The disclosed compounds are racemic isomers that contain the same amount of S and R structures of ulifoxacin salts; their stero configurations can be expressed as (±).

Kise Masahiro et al., Japanese Patent Application Publication No. 3-218383, disclosed the using laboratory HPLC with 3x50mm of ODS column to separate racemic 6-fluoro-1-methyl-4-oxo-7-(1-piperazinyl)-1H,4H-[1,3]thiazeto[3,2-a]quinoline-3-carboxylic acid and obtained about 80 mg of S and R structures, defined as L- and R-isomers. However, the disclosed separation method has limited capacity and high cost, and thus is not suitable for commercial production. L-isomers of the chemical, called (S)-6-fluoro-1-methyl-4-oxo-7-(1-piperazinyl)-1H,4*H*-[1,3]thiazeto[3,2-a]quinoline-3-carboxylic acid (or L-You Li Sha Xing), structure type 2, see below.

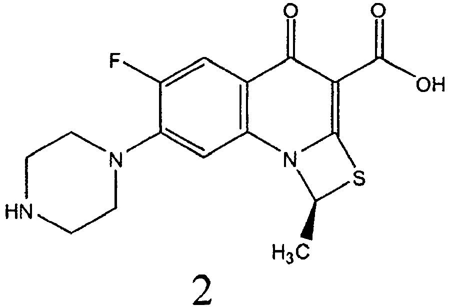

Jun Segawa, et al., Chem Pharm Bull (Tokyo), 1995 Jul; 43 (7): 1238, reported that they tested the in-vitro antibacterial activity of (S)-6-fluoro-1-methyl-4-oxo-7-(1-piperazinyl)-1H,4*H-*[1,3]thiazeto[3,2-a]quinoline-3-carboxylic acid and (R)-6-fluoro-1-methyl-4-oxo-7-(1-piperazinyl)-1H,4*H-*[1,3]thiazeto[3,2-a]quinoline-3-carboxylic acid showed that the activity of the S isomer is 2 to 8 times more potent that of the R isomer. However, the S isomer has low water solubility, which limits its clinical applications.

Although racemic ulifloxacin has an excellent antibacterial activity, it also has relatively high toxicity. Ishida S, Journal of Toxicological Sciences, 1996 Jun, 21 Suppl 1:131, reported the toxicity of racemic ulifloxacin NM394 to rats by intravenous injection. Male and female groups of Sprague-Dawley rats were, respectively, intravenously injected with 3, 10 and 30 mg/kg doses of NM394. For a period of four weeks, the rats injected with 10 and 30 mg/kg doses have showed significant increase in water consumption and urinary amount. Crystalline substance and epithelial cells were found in urinary precipitation. For the group of rats injected with 30mg/kg dose, their urea became cloudy. The rats injected with 10 and 30 mg/kg doses showed reduced serum γ-globulin. The groups of mice injected with 10 and 30 mg/kg doses have increased blood urea nitrogen and creatinine, indicating their kidney function was damaged. In addition, the rats injected with 10 and 30 mg/kg doses showed pathological changes such as tubular nephropathy, and crystalline material was found. The rats injected with 30 mg/kg dose showed increased weights of kidney and appendix. The 3 mg/kg dose group did show significant problem; thus for rats, the NOAEL (no observed adverse effect level) dose of NM394 should be 3mg/kg.

Shimazu H et al., Journal of Toxicological Sciences, 1996, Jun, 21 Suppl 1:33, reported the intravenous injection of NM394 caused rats to suffer from convulsions, difficult breathing, injection site redness, swelling, and necrosis, and caused mice to suffer from losing weight, testicular atrophy, and visible pulmonary congestion.

The above references indicate that racemic ulifloxacin has high irritation and toxicity, and thus it is of no clinical significance.

### DESCRIPTION OF THE INVENTION

The objective of the invention is to provide an optically active quinolone compound which can be used as an anti-infection medicine. The compound is readily soluble in water and has a higher antibacterial activity than NM394. It has low toxicity and low irritability to muscle and skin. It has low side effects, broad antibacterial spectrum, and is more suitable for clinic uses.

The technical scheme of the invention is to provide an anti-infection drug, which is an optically active quinolone compound having the following general structure:

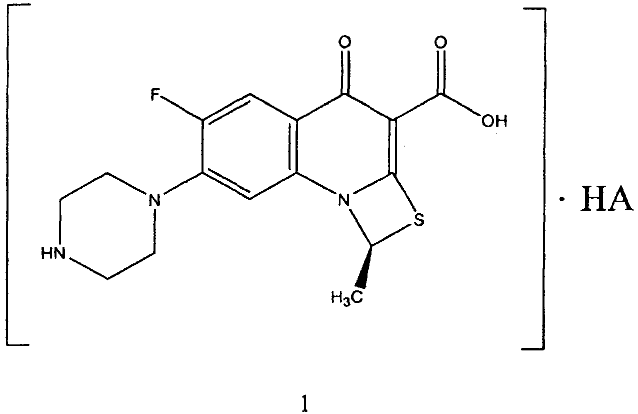

In the above structure, HA is an organic acid which forms a pharmaceutically acceptable compound with (S)-6-fluoro-1-methyl-4-oxo-7-(1-piperazinyl)-1H,4*H-*[1,3]thiazeto[3,2-a]quinoline-3-carboxylic acid.

In particular, S-ulifoxacin is isolated from racemic ulifloxacin, which then forms pharmaceutically acceptable compound with organic or inorganic acids.

**Said** organic acids **is selected from the group consisting of** methylsulfonic acid, lactic acid, glutamic acid, gluconic acid **and** aspartic acid. Suitable aspartic acid includes its DL, D, and L forms. Preferred acids include methylsulfonic acid, lactic acid, gluconic acid, gluconic acid, and aspartic acid.

The product formed by lactic acid and (S)-6-fluoro-1-methyl-4-oxo-7-(1-piperazinyl)-1H,4*H*-[1,3]thiazeto[3,2-a]quinoline-3-carboxylic acid is called (S)-6-fluoro-1-methyl-4-oxo-7-(1-piperazinyl)-1H,4*H*-[1,3]thiazeto[3,2-a]quinoline-3-carboxylic acid lactate. The product has rotation [α]²⁰_{D} from-112.5 to -118.2°; IR 1698 cm⁻¹, 1629 cm⁻¹, 1605 cm⁻¹, 1501 cm⁻¹, 1396 cm⁻¹, and 1257 cm⁻¹.

The product formed by methylsulfonic acid and (S)-6-fluoro-1-methyl-4-oxo-7-(1-piperazinyl)-1H,4*H*-[1,3]thiazeto[3,2-a]quinoline-3-carboxylic acid is called (S)-6-fluoro-1-methyl-4-oxo-7-(1-piperazinyl)-1H,4*H*-[1,3]thiazeto[3,2-a]quinoline-3-carboxylic acid mesylate. The product has rotation [α]²⁰_{D} from - 106.6 to -112.6°; IR 1707 cm⁻¹, 1629 cm⁻¹, 1602 cm⁻¹, and 1501 cm⁻¹.

The product formed by gluconic acid is used and (S)-6-fluoro-1-methyl-4-oxo-7-(1-piperazinyl)-1H,4*H*-[1,3]thiazeto[3,2-a]quinoline-3-carboxylic acid is called (S)-6-fluoro-1-methyl-4-oxo-7-(1-piperazinyl)-1H,4*H*-[1,3]thiazeto[3,2-a]quinoline-3-carboxylic acid gluconate. The product has IR 1695 cm⁻¹, 1629 cm⁻¹, 1601 cm⁻¹, and 1503 cm⁻¹.

The product formed by glutamic acid is used and (S)-6-fluoro-1-methyl-4-oxo-7-(1-piperazinyl)-1H,4*H*-[1,3]thiazeto[3,2-a]quinoline-3-carboxylic acid is called (S)-6-fluoro-1-methyl-4-oxo-7-(1-piperazinyl)-1H,4*H*-[1,3]thiazeto[3,2-a]quinoline-3-carboxylic acid glutamate. The product has IR 1628 cm⁻¹, 1603 cm⁻¹, 1499 cm⁻¹, 1457 cm⁻¹, 1397 cm⁻¹, 1257 cm⁻¹.

The product formed by aspartic acid and (S)-6-fluoro-1-methyl-4-oxo-7-(1-piperazinyl)-1H,4*H*-[1,3]thiazeto[3,2-a]quinoline-3-carboxylic acid is called (S)-6-fluoro-1-methyl-4-oxo-7-(1-piperazinyl)-1H,4*H*-[1,3]thiazeto[3,2-a]quinoline-3-carboxylic acid aspartate. The product has IR 1695 cm⁻¹, 1628 cm⁻¹, 1602 cm⁻¹, and 1499 cm⁻¹.

According to the invention, the compound 1 is obtained by the following method. Reacting D-tartrate solution in DMSO with (1)-6-fluoro-1-methyl-4-oxo-7-(1-piperazinyl)-1H,4H-[1,3]thiazeto[3,2-a]quinoline-3-carboxylic acid solution in DMSO yields a tartrate salt precipitate. Hydrolysis of the tartrate salt with NaOH solution yields (S)-6-fluoro-1-methyl-4-oxo-7-(1-piperazinyl)-1H,4*H*-[1,3]thiazeto[3,2-a]quinoline-3-carboxylic acid.

At 0 to 60°C, an HA solution having a given concentration was prepared; to the solution was then added (S)-6-fluoro-1-methyl-4-oxo-7-(1-piperazinyl)-1H,4*H*-[1,3]thiazeto[3,2-a]quinoline-3-carboxylic acid under agitation. The reaction continues for 1 to 6 hours until the reactor contents become clear and no solid substance was observed. Active carbon black (5%) was added to the reaction solution and the mixtires was then filtered. An organic solvent (1 to 100 times) was added to precipitate the product. The product was filtered and dried to yield compound 1. The above-mentioned organic solvent was selected from methanol, ethanol, isopropyl alcohol, acetone, tetrahydrofuran, or a mixture thereof. The feed ratio of (S)-6-fluoro-1-methyl-4-oxo-7-(1-piperazinyl)-1H,4*H*-[1,3]thiazeto[3,2-a]quinoline-3-carboxylic acid to HA is 1:0.8 to 1.5. HA is any acid described above; according to this method a corresponding compound is obtained.

The invention also provides anti-infection compositions which comprise the optical active, fluorine-containing quinolone compound 1 as an active ingredient and one or more pharmaceutically acceptable conventional carriers. For instance, the anti-infection composition comprises conventional accessories so that they can be converted to oral dosage forms such as medicinal tablets, capsules (including sustained-release and controlled-release formulations), powder, granular agent of solid, or to non-gastrointestinal delivery forms, such as injection agent.

The effectiveness of the invention:

The compound of the invention has stable properties. Compared to ulifloxacin, it has improved water solubility and reduced pH value in an aqueous solution. It is readily soluble in water; it has high antibacterial activity, low nephrotoxicity, and no irritability to muscle and skin. In the experiments, the level of no adverse drug effect is 30 mg/kg, which is 10 times higher than that of ulifloxacin. It has low side effects, broad antibacterial spectrum, and its activity is 1-3 times higher than the DL-ulifloxacin.

The following examples further illustrate the technical scheme and effectiveness of the invention. However, these examples do not limit the scope of the invention.

### EXAMPLE 1

### Preparation of (S)-6-fluoro-1-methyl-4-oxo-7-(1-piperazinyl)-1H,4H-[1,3]thiazeto[3,2-a]quinoline-3-carboxylic acid

Racemic ulifloxacin (105 g) was dissolved in DMSO (1500 mL) D-tartrate (27 g) solution in DMSO (405 mL) was added to the racemic ulifoxacin solution with agitation. Cloudiness and precipitation appear. After 20 hours of agitation at an ambient temperature, the mixture was filtered. The solid was dried under vacuum to yield 86 g of solid. The solid was recrystallized in DMSO to yield 37 g of (S)-6-fluoro-1-methyl-4-oxo-7-(1-piperazinyl)-1H,4*H-*[1,3]thiazeto[3,2-a] quinoline-3-carboxylic acid-D-tartrate salt; the elemental analysis indicates: C 49.08%, H 5.06%, N 9.50%, and S 7.44% (corresponding to: C₁₆H₁₆FN₃O₃S•1/2C₄H₆O₆•H₂O, calculated value: C 48.86%, H 4.78, N 9.50%, and S 7.25%). The salt was dispersed in water and the dispersion was neutralized with 2% NaOH aqueous solution to a pH value of 7 to 8. The precipitate is filtered and dried to yield 24.5 g of (S)-6-fluoro-1-methyl-4-oxo-7-(1-piperazinyl)-1H,4H-[1,3]thiazeto[3,2-a]quinoline-3-carboxylic acid. It has a rotation [α]²⁰_{D} = -143.4° (c = 0.15, 0.1 mol/L NaOH), ¹H-NMR (DMSO-d6) δ 2.11 (3H, d, j=6.2 Hz), 2.85∼3.20 (8H, m), 6.40 (1H, q, j=6.2 Hz), 6.89 (1H, d, j=7.4Hz), 7.79 (1H, d, j=13.9 Hz), optical purity e.e.>95%.

### EXAMPLE 2

### Preparation of (R)-6-fluoro-1-methyl-4-oxo-7-(1-piperazinyl)-1H,4H-[1,3]thiazeto[3,2-a]quinoline-3-carboxylic acid (Compound 3)

Racemic ulifloxacin (105 g) was dissolved in DMSO (1500 mL). L-tartrate (27 g) solution in DMSO (405 mL) was added to the racemic ulifoxacin solution with agitation. Cloudiness and precipitation appear. After 20 hours of agitation at an ambient temperature, the mixture was filtered and dried under vacuum to yield 82 g of solid. The solid was recrystallized in DMSO to yield 34 g of (R)-6-fluoro-1-methyl-4-oxo-7-(1-piperazinyl)-1H,4H-[1,3]thiazeto[3,2-a]quinoline-3-carboxylic acid-L-tartrate salt. The salt was dispersed in water and the dispersion was neutralized with 2% NaOH aqueous solution to a pH value of 7 to 8. The precipitate was filtered and dried to yield 22 g of (R)-6-fluoro-1-methyl-4-oxo-7-(1-piperazinyl)-1H,4H-[1,3]thiazeto[3,2-a]quinoline-3-carboxylic acid. It has specific rotation [α]²⁰D = +139.2° (c=0.15, 0,1 mol/L NaOH), optical purity e.e>95%.

### EXAMPLE 3

### Preparation of (S)-6-fluoro-1-methyl-4-oxo-7-(1-piperazinyl)-1H,4H-[1,3]thiazeto[3,2-a]quinoline-3-carboxylic acid lactate (Compound 4).

At 20°C, a flask was charged with 30 mL of water, 1.6 g of lactic acid, and then with 5 g of (S)-6-fluoro-1-methyl-4-oxo-7-(1-piperazinyl)-1H,4*H-*[1,3]thiazeto[3,2-a]quinoline-3-carboxylic acid under agitation. After 60 minutes of agitation, an essentially clear liquid was obtained. To the liquid was added 5% of active carbon to discolor for 30 minutes. The mixture was filtered. To the filtrate was added 200 mL of anhydrous ethanol to precipitate the solid. After 2 hours of agitation, the mixture was filtered; the solid cake was crushed and dried at 50°C under vacuum to yield 3.6 g of (S)-6-fluoro-1-methyl-4-oxo-7-(1-piperazinyl)-1H,4H-[1,3]thiazeto[3,2-a]quinoline-3-carboxylic acid lactate.[α]²⁰_{D} = -116.5 (c=1.0, H₂O); ¹H-NMR (D₂O)δ 1.30 (3H, d, j=6.4Hz),2.06 (3H, d, j=6.0Hz), 3.44 to 3.51 (8H, m), 4.10 (1H, q, j=10.4Hz), 6.07 (1H, q, j=6.4Hz), 6.47 (1H, d, j=6.8Hz), 7.24 (1H, d, j=13.2Hz), IR 1698 cm⁻¹, 1629 cm⁻¹, 1605 cm⁻¹, 1501 cm⁻¹, 1396 cm⁻¹, and 1257 cm⁻¹.

### EXAMPLE 4

### Preparation of (S)-6-fluoro-1-methyl-4-oxo-7-(1-piperazinyl)-1H,4H-[1,3]thiazeto[3,2-a]quinoline-3-carboxylic acid mesylate (Compound 5)

At 20°C, a flask was charged with 30 mL of water, 2.1 g of methylsulfonic acid, and then with 5 g of (S)-6-fluoro-1-methyl-4-oxo-7-(1-piperazinyl)-1H,4*H-*[1,3]thiazeto[3,2-a]quinoline-3-carboxylic acid under agitation. After 60 minutes of agitation, an essentially clear liquid was obtained. To the liquid was added 5% of active carbon to discolor for 30 minutes. The mixture was filtered. To the filtrate was added 200 mL of anhydrous ethanol under agitation for 1 hour to precipitate the solid. After additional 2 hours of agitation, the mixture was filtered; the solid was dried at 60°C under vacuum to yield 4.0 g of (S)-6-fluoro-1-methyl-4-oxo-7-(1-piperazinyl)-1H,4H-[1,3]thiazeto[3,2-a]quinoline-3-carboxylic acid mesylate. [α]²⁰_{D} = -109.4° (c=1.0, H₂O), IR 1707 cm⁻¹, 1629 cm⁻¹, 1602 cm⁻¹, 1501 cm⁻¹; ¹H-NMR (D₂O) δ 2.00 (3H, d, j=6Hz), 2.73 (3H, s), 3.39∼3.45 (8H, m), 6.05 (1H, q, j=6Hz), 6.42 (1H, d, j=5.2Hz), 7.21 (1H, d, j=13.2Hz); elemental analysis: C 45.32%, H 4.85%, N 9.25%, (C₁₆H₁₆FN₃O₃S•CH₄O₃S•1/2H₂O; calculated value: C 44.93%, H 4.66% and N 9.25%).

### EXAMPLE 5

### Preparation of (S)-6-fluoro-1-methyl-4-oxo-7-(1-piperazinyl)-1H,4H-[1,3]thiazeto[3,2-a]quinoline-3-carboxylic acid gluconate (Compound 6)

At 20°C, a flask was charged with 30 mL of water, 6.8 mL of 50% gluconic acid aqueous solution, and then with 5 g of (S)-6-fluoro-1-methyl-4-oxo-7-(1-piperazinyl)-1H,4*H*-[1,3]thiazeto [3,2-a]quinoline-3-carboxylic acid under agitation. After 60 minutes of agitation, an essentially clear liquid was obtained. To the liquid was added 5% of active carbon to discolor for 30 minutes. The mixture was filtered. To the filtrate was added 200 mL of anhydrous ethanol under agitation for 1 hour to precipitate the solid. After additional 2 hours of agitation, the mixture was filtered; the solid was dried at 50°C under vacuum to yield 4.0 g of (S)-6-fluoro-1-methyl-4-oxo-7 (1-piperazinyl)-1H,4*H*-[1,3]thiazeto[3,2-a]quinoline-3-carboxylic acid gluconate.

Analysis results: IR 1695 cm⁻¹, 1629 cm⁻¹, 1601 cm⁻¹, 1503 cm⁻¹ ; ¹H-NMR (DMSO-d6) δ 2.12 (3H, s), 6.39 (1H, s), 6.95 (1H, s), and 7.80 (1H, d, j=13.6Hz).

### EXAMPLE 6

### Preparation of (S)-6-fluoro-1-methyl-4-oxo-7-(1-piperazinyl)-1H,4H-[1,3]thiazeto[3,2-a]quinoline-3-carboxylic acid aspartate (Compound 7)

At 20°C, a flask was charged with 30 mL of water, 20 g of aspartic acid, and then with 5 g of (S)-6-fluoro-1-methyl-4-oxo-7-(1-piperazinyl)-1H,4*H-*[1,3]thiazeto[3,2-a]quinoline-3-carboxylic acid under agitation. After 60 minutes of agitation, an essentially clear liquid was obtained. To the liquid was added 5% of active carbon to discolor for 30 minutes. The mixture was filtered. To the filtrate was added 200 mL of anhydrous ethanol under agitation for 1 hour to precipitate the solid. After additional 2 hours of agitation, the mixture was filtered; the solid was dried at 50°C under vacuum to yield 3.7 g of (S)-6-fluoro-1-methyl-4-oxo-7-(1-piperazinyl)-1H,4H-[1,3]thiazeto[3,2-a]quinoline-3-carboxylic acid aspartate. IR 1695 cm⁻¹, 1628 cm⁻¹, 1602 cm⁻¹, 1499 cm⁻¹; ¹H-NMR (D₂O) δ 2.13 (2H, m), 2.70∼2.89 (3H, s), 3.50-3.57 (8H, m), 3.93 (1H, q, j=4.8Hz), 6.16 (1H, s), 6.57 (1H, s), and 7.35 (1H, d, j=12.8Hz).

### EXAMPLE 7

### Preparation of (S)-6-fluoro-f-methyl-4-oxo-7-(1-piperazinyl)-1H,4H-[1,3]thiazeto[3,2-a]quinoline-3-carboxylic acid glutamate (Compound 8)

At 20°C, a flask was charged with 30 mL of water, 2.8 mL g of glutamic acid, and then with 5g of (S)-6-fluoro-1-methyl-4-oxo-7-(1-piperazinyl)-1H,4*H-*[1,3]thiazeto[3,2-a]quinoline-3-carboxylic acid under agitation. After 60 minutes of agitation, an essentially clear liquid was obtained. To the liquid is added 5% of active carbon to discolor for 30 minutes. The mixture was filtered. To the filtrate was added 200 mL of anhydrous ethanol under agitation for 1 hour to precipitate the solid. After additional 2 hours of agitation, the mixture was filtered; the solid was dried at 50°C under vacuum to yield 3.6 g of (S)-6-fluoro-1-methyl-4-oxo-7-(1-piperazinyl)-1H,4H-[1,3]thiazeto[3,2-a]quinoline-3-carboxylic acid glutamate. ¹H-NMR (D₂O) δ 2.1-2.5 (6H, m), 2.40∼2.47 (3H, m), 3.49∼3.59 (8H, m), 3.80 (1H, q, j=1.6Hz), 6.09 (1H, s), 6.49 (1H, s), and 7.20 (1H, d, j=13.2Hz).

### EXAMPLE 8

### Preparation of (R)- 6-fluoro-1-methyl-4-oxo-7-(1-piperazinyl)-1H,4H-[1,3]thiazeto[3,2-a]quinoline-3-carboxylic acid mesylate (Compound 9)

At 20°C, a flask was charged with 30 mL of water, 2 1 mL g of methylsulfonic acid, and then with 5g of (R)-6-fluoro-1-methyl-4-oxo-7-(1-piperazinyl)-1H,4H-[1,3]thiazeto[3,2-a]quinoline-3-carboxylic acid under agitation. After 60 minutes of agitation, an essentially clear liquid was obtained. To the liquid was added 5% of active carbon to discolor for 30 minutes. The mixture was filtered. To the filtrate was added 200 mL of anhydrous ethanol under agitation for 1 hour to precipitate the solid. After additional 2 hours of agitation, the mixture was filtered; the solid was dried at 60°C under vacuum to yield 4.0 g of (R)-6-fluoro-1-methyl-4-oxo-7-(1-piperazinyl)-1H,4H-[1,3]thiazeto[3,2-a]quinoline-3-carboxylic acid mesylate. [α]²⁰_{D} = +107.1°, (c=1.0, H₂O), IR 1705 cm⁻¹, 1628 cm⁻¹, 1602 cm⁻¹, 1501 cm⁻¹; ¹H-NMR (D₂O) δ 2.02 (3H, d, j=3.6Hz), 2.73 (3H, s), 3.40∼3.48 (8H, m), 6.09 (1H, s), 6.46 (1H, d, j=5.2Hz), 7.21 (1H, d, j=13.2Hz); elemental analysis C 44.60%, H 5.01%, N 9.08%, (C₁₆H₁₆FN₃O₃S•CH₄O₃S•1/2H₂O; calculated C 44.93%, H 4.66% and N 9.25%).

### EXAMPLE 9

### Preparation of (R)-6-fluoro-1-methyl-4-oxo-7-(1-piperazinyl)-1H,4H-[1,3]thiazeto[3,2-a]quinoline-3-carboxylic acid gluconate (Compound 10)

At 20°C, a flask was charged with 30 mL of water, 6.8 mL g of 50% gluconic acid aqueous solution, and then with 5 g of (R)-6-fluoro-1-methyl-4-oxo-7-(1-piperazinyl)-1H,4*H-*[1,3]thiazeto[3,2-a]quinoline-3-carboxylic acid under agitation. After 60 minutes of agitation, an essentially clear liquid was obtained. To the liquid was added 5% of active carbon to discolor for 30 minutes. The mixture was filtered. To the filtrate was added 200 mL of anhydrous ethanol under agitation for 1 hour to precipitate the solid. After additional 2 hours of agitation, the mixture was filtered; the solid was dried at 50°C under vacuum to yield 4.0 g of (R)-6-fluoro-1-methyl-4-oxo-7-(1-piperazinyl)-1H,4*H*-[1,3]thiazeto[3,2-a]quinoline-3-carboxylic acid.

### EXAMPLE 10

### Preparation of (R)-6-fluoro-1-methyl-4-oxo-7-(1-piperazinyl)-1H,4H-[1,3]thiazeto[3,2-a]quinoline-3-carboxylic acid aspartate (Compound 11).

At 20°C, a flask was charged with 30 mL of water, 20 g of aspartic acid, and then with 5 g of (R)-6-fluoro-1-methyl-4-oxo-7-(1-piperazinyl)-1H,4H-[1,3]thiazeto[3,2-a]quinoline-3-carboxylic acid under agitation. After 60 minutes of agitation, an essentially clear liquid was obtained. To the liquid was added 5% of active carbon to discolor for 30 minutes. The mixture was filtered. To the filtrate was added 200 mL of anhydrous ethanol under agitation for 1 hour to precipitate the solid. After additional 2 hours of agitation, the mixture was filtered; the solid was dried at 60°C under vacuum to yield 3.7 g of (R)-6-fluoro-1-methyl-4-oxo-7-(1-piperazinyl)-1H,4*H-*[1,3]thiazeto[3,2-a]quinoline-3-carboxylic acid aspirate.

### EXAMPLE 11

### Preparation of (R)-6-fluoro-1-methyl-4-oxo-7-(1-piperazinyl)-1H,4H-[1,3]thiazeto[3,2-a]quinoline-3-carboxylic acid glutamate (Compound 12).

At 20°C, a flask was charged with 30 mL of water, 2.8 mL of glutamic acid, and then with 5g of (R)-6-fluoro-1-methyl-4-oxo-7-(1-piperazinyl)-1H,4*H-*[1,3]thiazeto[3,2-a]quinoline-3-carboxylic acid under agitation. After 60 minutes of agitation, an essentially clear liquid was obtained. To the liquid was added 5% of active carbon to discolor for 30 minutes. The mixture was filtered. To the filtrate was added 200 mL of anhydrous ethanol under agitation for 1 hour to precipitate the solid. After additional 2 hours of agitation, the mixture was filtered; the solid was dried at 50°C under vacuum to yield 3.6 g of (R)-6-fluoro-1-methyl-4-oxo-7-(1-piperazinyl)-1H,4*H-*[1,3]thiazeto[3,2-a]quinoline-3-carboxylic acid glutamate.

### EXAMPLE 12

### Preparation of (R)-6-fluoro-1-methyl-4-oxo-7-(1-piperazinyl)-1H,4H-[1,3]thiazeto[3,2-a]quinoline-3-carboxylic acid lactate (Compound 13)

At 20°C, a flask was charged with 30 mL of water, 1.6 g of lactic acid, and then with 5 g of (R)-6-fluoro-1-methyl-4-oxo-7-(1-piperazinyl)-1H,4*H-*[1,3]thiazeto[3,2-a]quinoline-3-carboxylic acid under agitation. After 60 minutes of agitation, an essentially clear liquid was obtained. To the liquid was added 5% of active carbon to discolor for 30 minutes. The mixture was filtered. To the filtrate was added 200 mL of anhydrous ethanol under agitation for 1 hour to precipitate the solid. After additional 2 hours of agitation, the mixture was filtered; the solid cake was crushed and dried at 50°C under vacuum to yield 3.6 g of (R)-6-fluoro-1-methyl-4-oxo-7-(1-piperazinyl)-1H,4*H*-[1,3]thiazeto[3,2-a]quinoline-3-carboxylic acid lactate.

### Reference EXAMPLE 13

### Preparation of (S)-6-fluoro-1-methyl-4-oxo-7-(1-piperazinyl)-1H,4H-[1,3]thiazeto[3,2-a]quinoline-3-carboxylic acid chloride

At 20°C, a flask was charged with 30 mL of water, 1.6 g of lactic acid, and then with 5 g of (S)-6-fluoro-1-methyl-4-oxo-7-(1-piperazinyl)-1H,4*H-*[1,3]thiazeto[3,2-a]quinoline-3-carboxylic acid under agitation. After 60 minutes of agitation, an essentially clear liquid was obtained. To the liquid was added 5% of active carbon to discolor for 30 minutes. The mixture was filtered. To the filtrate was dropwise added 1.8 mL of 36% hydrochloric acid under agitation to precipitate the solid. After additional 2 hours of agitation, the mixture was filtered; the solid cake was crushed, washed with water and dried at 50°C under vacuum to yield 4.6 g of (S)-6-fluoro-1-methyl-4-oxo-7-(1-piperazinyl)-1H,4*H*-[1,3]thiazeto[3,2-a]quinoline-3-carboxylic acid chloride.

### Reference EXAMPLE 14

### Preparation of (S)-6-fluoro-1-methyl-4-oxo-7-(1-piperazinyl)-1H,4H-[1,3]thiazeto[3,2-a]quinoline-3-carboxylic acid sulfate

At 20°C, a flask was charged with 30 mL of water, 1.6 g of lactic acid, and then with 5 g of (S)-6-fluoro-1-methyl-4-oxo-7-(1-piperazinyl)-1H,4*H-*[1,3]thiazeto[3,2-a]quinoline-3-carboxylic acid under agitation. After 60 minutes of agitation, an essentially clear liquid was obtained. To the liquid was added 5% of active carbon to discolor for 30 minutes. The mixture was filtered. To the filtrate was dropwise added 1.4 mL of 98% sulfuric acid under agitation to precipitate the solid. After additional 2 hours of agitation, the mixture was filtered; the solid cake was crushed, washed with water, and dried at 50°C under vacuum to yield 4.8 g of (S)-6-fluoro-1-methyl-4-oxo-7-(1-piperazinyl)-1H,4*H*-[1,3]thiazeto[3,2-a]quinoline-3-carboxylic acid sulfate.

### EXAMPLE 15

### Solubility Test

Three lots of mesylates and three lots of lactates, respectively, are subjected to preliminary tests for maximum solubility in water. At ambient temperature (25°C), 0.2 g of sample was dissolved in 1 mL of water. To the solution was added 0.1 g of the sample each time until the sample can no longer be dissolved. The amount of sample dissolved is the maximum solubility. The value is calculated by the average of two tests; the results are listed in the following table.

| Isomers | Compounds | | Water Solubility mg/mL |
|---|---|---|---|
| Racemic (±) | Mesylate | Prepared according to Chinese Pat. Appl. No. 200610033028.0 | 340 |
| | Lactate | Prepared according to Chinese Pat. Appl. No. 200610033042.0 | 106 |
| Levorotary (S) | Mesylate | Compound 5 of Example 4 | 490 |
| | Lactate | Compound 4 of Example 3 | 70 |
| Dextrorotatory (L) | Mesylate | Compound 9 of Example 8 | 501 |
| | Lactate | Compound 13 of Example 12 | 116 |

The results show that the mesylates have better water solubility than lactates, and levorotary mesylates have better solubility than dextrorotatory mesylates.

### EXAMPLE 16

### In-vitro antibacterial tests

Test method: the agar dilution method was used for the minimum inhibitory concentration (MIC) determination. The antibacterial chemicals are mixed with given amounts of agar in various concentrations and the mixtures are made into solid plates. Each plate has the antibacterial concentration two times higher than the next plate. Bacteria species are then added to the surface of the plates, cultured, and observed for their growth. According to the U.S. Committee for Clinical Laboratory Standards (NCCIS) standard, the test plates are placed in dark, non-reflective surfaces to determine the end point of the bacteria growth; the lowest concentration at which no bacteria growth was observed was reported as the MIC. In all the experiments, the MIC of the quality control strains meets the quality control standards of the NCCIS.

If the bacteria species grow on the tested plates which has the concentration higher than the end point, or the bacteria species grow on a plate which has a higher concentration while does not grow on the plate which has a lower concentration, the purity of the cultures will be double-checked or the tests are repeated.

### Test Samples:

Ciprofloxacin, levofloxacin, and (±) ulifloxacin from the market are used as controls.
(S)-6-fluoro-1-methyl-4-oxo-7-(1-piperazinyl)-1H,4H-[1,3]thiazeto[3,2-a]quinoline-3-carbo xylic acid mesylate (Compound 5);
(S)-6-fluoro-1-methyl-4-oxo-7-(1-piperazinyl)-1H,4H-[1,3]thiazeto[3,2-a]quinoline-3-carboxylic acid lactate (Compound 4);
(S)-6-fluoro-1-methyl-4-oxo-7-(1-piperazinyl)-1H,4H-[1,3]thiazeto[3,2-a]quinoline-3-carboxylic acid glutamate (Compound 8);
(S)-6-fluoro-1-methyl-4-oxo-7-(1-piperazinyl)-1H,4H-[1,3]thiazeto[3,2-a]quinoline-3-carboxylic acid gluconate (Compound 6); and
(S)-6-fluoro-1-methyl-4-oxo-7-(1-piperazinyl)-1H,4H-[1,3]thiazeto[3,2-a]quinoline-3-carboxylic acid aspartate (Compound 7) are prepared according to the examples of this invention.
(±)-6-fluoro-1-methyl-4-oxo-7-(1-piperazinyl)-1H,4H-[1,3]thiazeto[3,2-a]quinoline-3-carboxylic acid lactate was prepared according to the examples of Chinese Pat. Appl. No. 200610033042.0.
(+)-6-fluoro-1-methyl-4-oxo-7-(1-piperazinyl)-1H,4H-[1,3]thiazeto[3,2-a]quinoline-3-carboxylic acid mesylate was prepared according to the examples of Chinese Pat. Appl. No. 200610033028.0.
(+)-6-fluoro-1-methyl-4-oxo-7-(1-piperazinyl)-1H,4H-[1,3]thiazeto[3,2-a]quinoline-3-carboxylic acid gluconate was prepared according to the examples of Chinese Pat. Appl. No. 200610033033.1.
(+)-6-fluoro-1-methyl-4-oxo-7-(1-piperazinyl)-1H,4H-[1,3]thiazeto[3,2-a]quinoline-3-carboxylic acid aspartate was prepared according to the examples of Chinese Pat. Appl. No. 200610033034.6.
(±)-6-fluoro-1-methyl-4-oxo-7-(1-piperazinyl)-1H,4H-[1,3]thiazeto[3,2-a]quinoline-3-carboxylic acid glutamate was prepared according to the examples of Chinese Pat. Appl. No. 200610033039.9.

The test bacterial strains are the standard quality control test strains of Klebsiella pneumoniae strains (Strains No. CMCC 46114-8), Pseudomonas aeruginosa (Strains No. ATCC 27853), Escherichia coli (Strains No. ATCC25922), and Staphylococcus aureus cocci (Strains No. ATCC25925). Other bacteria include those isolated from sputum, throat swab or urine of patients with acute bacterial respiratory tract infection or urinary tract infection and identified by the hospitals.

(S)-6-fluoro-1-methyl-4-oxo-7-(1-piperazinyl)-1H,4H-[1,3]thiazeto[3,2-a]quinoline-3-carboxylic acid lactate and other test samples, equivalent to 96 mg of ulifloxacin, are dissolved in sterilized distilled water to make up 50 mL of each solution. HCl levofloxacin injection and NaCl ciprofloxacin lactate injection are diluted with sterilized distilled water to a concentration of 1920 µg/mL. The in-vitro antibacterial test results are listed in Table 1.

**Table 1. In-vitro MIC (µg/mL)**

| Sample Name | Stero-structure | Bacteria tested | | | | | | | |
|---|---|---|---|---|---|---|---|---|---|
| | | Pseudomonas aeruginosa | Staphylococc us aureus | Enterococcus faecalis | Streptococcus pneumoniae | Escherichia coli | Klebsiella pneumoniae | Enterobacter cloacae | Vibrio cholerae |
| Levofloxacin | (S) | 2 | 0.5 | - | - | 0.0313 | 0.0125 | - | - |
| Ciprofloxacln | (±) | 0.5 | 0.5∼1 | - | - | 0.0313 | 0.0313∼ 0.0625 | - | - |
| Compound 2 | (S) | 0.177 | 0.177 | - | 0.025 | 0.0125 | - | 0.5 | - |
| Compound 3 | (R) | 2 | 2.828 | - | 0.25 | 0.225 | - | 4 | - |
| Ullfloxacln | (±) | 0.5 | 0.5 | - | 0.031 | 0.08 | - | 1 | - |
| Ulifloxacin lactate | (S) | 0.25 | 0.125 | 0.5 | 0.02 | 0.01 | - | 0.5 | - |
| | (R) | 1 | 1 | 4 | 0.125 | 0.25 | - | 4 | - |
| | (±) | 0.5 | 0.25 | 2 | 0.1 | 0.125 | - | 1.25 | - |
| Ulifloxacin mesylate | (S) | 0.25 | 0.25 | 0.2 | 0.01 | 0.0125 | 0.02 | 0.5 | 0.02 |
| | (R) | 1 | 2 | 4 | 0.125 | 0.225 | 0.5 | 4 | 2 |
| | (±) | 0.5 | 0.5 | 1.5 | 0.088 | 0.063 | 0.2 | 1 | 0.5 |
| Ulifloxacin gluconate | (S) | 0.25 | 0.25 | - | - | 0.01 | 0.02 | - | 0.02 |
| | (R) | 1 | 1 | - | - | 0.25 | 0.5 | - | 2 |
| | (±) | 0.5 | 0.5 | - | - | 0.05 | 0.2 | - | 0.5 |
| Ulifloxacin aspartate | (S) | 0.25 | 0.25 | - | - | 0.02 | - | - | - |
| | (R) | 1 | 2 | - | - | 0.25 | - | - | - |
| | (±) | 0.5 | 0.5 | - | - | 0.1 | - | - | - |
| Ullfloxacln glutamate | (S) | - | 0.25 | - | 0.125 | 0.01 | - | - | - |
| | (R) | - | 2 | - | 0.5 | 0.5 | - | - | - |
| | (±) | - | 0.5 | - | 0.2 | 0.125 | - | - | - |

The results indicate that the antibacterial activity of the S-ulifloxacin is 3 to 10 times of the R-ulifloxacin and two or more times of racemic ulifloxacin. Compounds 2, 4, 5, 6, 7, and 8 have relatively strong antibacterial activity against Klebsiella pneumoniae, Pseudomonas aeruginosa, Staphylococcus aureus, Escherichia coli, Enterococcus faecalis, Streptococcus pneumoniae, Enterobacter cloacae, and Vibrio cholerae. The antibacterial activities of these compounds against Klebsiella pneumoniae and Escherichia coli are stronger than those against Pseudomonas aeruginosa, Enterococcus faecalis, and Staphylococcus aureus. The in-vitro antibacterial activities of the S-ulifloxacin serial products against Pseudomonas aeruginosa are stronger than that of Levofloxacin, but close to that of Ciprofloxacin; their in-vitro antibacterial activities against Klebsiella pneumoniae, Escherichia coli, and Staphylococcus aureus are slightly better or close to those of Ciprofloxacin and Levofloxacin. If the concentrations of the ulifloxacin serial products are calculated based on their equivalents to NM394, the antibacterial activities of compounds 4, 5, and 6 against the above four bacteria are slightly stronger than those of compounds 7 and 8.

### EXAMPLE 17

### Vascular irritation tests

Sixteen New Zealand rabbits are divided into 4 groups: a control group and three treatment groups for compounds 4, 5, and 6, respectively; each group has 4 rabbits. The rabbits of the treatment groups were vein-injected on their left ears with high doses of the test samples and on their right ears with low doses of the test samples. The rabbits of the control group were rein-injected on their both left and right ears with sodium chloride solution. The injections continued for 3 days with once a day. Two rabbits from each group were anatomized after 24 hours of each injection. The results indicate that 14 rabbit ears showed clear outline blood vessels, remained uniform thickness, and showed no significant changes. The pathological histology showed that rabbit ears had no toxicologically significant vascular changes. This indicates that compounds 4, 5, and 6 have no irritation to the in New Zealand rabbit ear vein and surrounding tissues.

### EXAMPLE 18

### Toxicity test

Compounds 4 and 5 were selected for the toxicity test. SD rats were administrated by intravenous injection of the compound samples and observed for toxicity reaction, severity, and reversibility of the damage to the main targeted organs. The dose without toxic reaction was thereby determined and the long term toxicity to SD rats was tested. One hundred forty SD rats were randomly divided into 7 groups depending on their body weight and sex; each group has 20 rats with 10 males and 10 females. Compounds 5 and 4 each sets 10, 30, and 60 mg/kg • bw (NM394 are calculated) three dose groups; and there was a blank control group. The rats were administrated by intravenous injection, once a day for consecutive 4 weeks. The recovery was monitored for 2 weeks after treatment.

Test groups and dose settings are:

As discussed above, the tests were divided into three dose groups and on blank control group; each group has 4 males and 4 females. The test groups and doses used are listed in Table 2.

**Table 2**

| | **Test groups and doses** | | |
|---|---|---|---|
| Group | Dose | Number of Rats | |
| | (mg /kg•bw) | Female | Male |
| Blank Control Group | 0 | 10 | 10 |
| Compound 5, Low Dose Group | 10 | 10 | 10 |
| Compound 5, Medium Dose Group | 30 | 10 | 10 |
| Compound 5, High Dose Group | 60 | 10 | 10 |
| Compound 4, Low Dose Group | 10 | 10 | 10 |
| Compound 4, Medium Dose Group | 30 | 10 | 10 |
| Compound 4, High Dose Group | 60 | 10 | 10 |

Drug preparation: the drugs were dissolved with a saline solution to the desired concentration before administration.
Administration: intravenous injection.
Delivery period: 28 days continuously.

### Observation and inspection:

Observation and inspection projects, targets and time: including the general state of observation (7), hematologic (16), biochemical blood test (18), urine (10) and pathological examination.

### Tested drugs:

Name: Compounds 4 and 5.

### Observation and inspection methods:

General observation: the appearance of signs, behavior, glands, respiratory and stool, daily observation and timely job records, discovering dead or dying animals, timely anatomical examination, food intake, and body weight once weekly.

Blood tests: abdominal aortic blood for hematology testing, in the 16 testing indicators, leukocyte classification using whole blood smear, Wright stain staining; prothrombin time (PT) with a 0.109 mmol/L of citrate Citrus medica sodium (with blood volume ratio of 1:9) anticoagulant, 3000r/min centrifugal 10min, taking with BE Thromotimer plasma coagulation analyzer (Germany) determination; other indicators to EDTA anticoagulant, using SWELAB AC920EO automated hematology analyzer (Sweden).

Biochemical blood tests: blood sampling method same s above, blood serum obtained by 3000r/min centrifugal 10min. In the 21 indicators the Na⁺, K⁺, Cluse EasyLyte Plus Na/K/Cl analyzer (USA) determination, the other indexes of blood with a Hitachi 7020 automatic biochemical analyzer (Japan) determination.

Urine tests: 16h urine collection, use CLINITEK 100 urine analyzer.

Pathology: after anesthesia, the abdominal aorta killed by blood letting. System anatomy and visual observation of changes in various organs were performed; organ weights were determined. Tissue was fixed in 10% neutral formalin; plates were made by conventional paraffin producers, HE staining, and light microscopy.

Statistical analysis: In the different groups, the body weight, hematology, blood chemistry, urine measurements, organ weights, organ coefficient were calculated for the mean ± standard deviation and t-test comparison between groups. Pathological abnormalities according to their incidence and severity were compared between groups.

### Test results:

### Observation of general conditions:

During the administration and recovery observation period, compared with the control group, the treatment group animals showed no abnormal changes in the body weight, food intake, appearance, behavior, gland secretion, respiratory conditions, and in stool.

### Hematology tests:

After 4 weeks of the drug administration, the female rats of the low dose groups and the medium dose groups showed slight decrease in leukocyte and slight increase in lymphocytes. However, the differences were insignificant (P> 0.05) compared with the rats of the control group, and there were no abnormal changes in other indicators. After 4 weeks of the drug administration, the female rats of the high dose groups showed slight decrease in leukocyte but significant increase in lymphocytes compared with the rats of the control group. After the recovery period, the Hematology indicators of all dose groups showed no abnormal changes.

### Blood biochemical tests:

The examinations for both after the 4 weeks of the drug administration and after the recovery period showed no abnormal changes in the blood biochemical indicators (P> 0.05), compared with the rats of the control group.

After 4 weeks of the drug administration, the male rats in the high dose groups showed significant increase in urea nitrogen and creatinine compared with the rats in the control group (P<0.05); other indicators showed no abnormal changes. After the recovery period, the rats in the high dose groups showed no abnormal changes in the blood biochemical indicators.

### Urea tests

After 4 weeks of the drug administration, the male rats of all dose groups showed significant decrease (P<0.05) in the urea pH value compared with the control group; the male rats of the high dose group also showed a small amount of phosphate crystals in urine; and there were no other abnormal changes. After the recovery period, the rats of all dose groups showed no abnormal changes in the urea tests.

### Pathological examination:

General pathology examination: compared with the control group, the male rats of the high dose groups, after 4 weeks of the drug administration, showed significant weight increase in both of the left and right kidneys (P <0.05) and significant increase in the adrenal weight coefficient (P <0.05); there were no other noticeable toxicological changes in the tissues, organs, organ weight, and organ coefficient.

Tissue microscopy: after 28 days of the continuous drug administration, two rats of the compound 5, high dose group showed mild cortical tubular dilatation, one rat showed kidney protein casts, and two rats (5 ♂, 7 ♂) showed crystals in renal tubules. After 28 days of the continuous drug administration, in the compound 4, high dose group, 3 rats showed mild cortical tubular dilatation, and one showed kidney protein casts. In the compound 4, high dose group, 1 rat showed mild interstitial inflammatory cell infiltration and 3 rats showed crystals in renal tubules. The microscopy of other organs and tissues in all dose groups showed no pathological changes of toxicological significance.

### Conclusion:

The above results indicate: for compounds 5 and 4, 28 days of continuous intravenous administration at a dose less than 30mg/kg•bw (calculated are NM394) caused no toxicologically significant changes to the rats; this dose was determined to be NOAEL (no observed adverse effect level) for the SD rats. When the continuous intravenous administration at a dose greater than 60mg/kg•bw, the SD rats showed toxicity reaction; the targeted organ was the kidney; the blood-sensitive indicators are the blood creatinine and blood urea nitrogen; and the urine-sensitive indicators are phosphate crystals and the pH value. It can be hypothesized that compounds 5 and 4 in clinical application shall be closely monitored for changes in renal function and urine.

### INDUSTRIAL APPLICABILITY

The invention provides a fluorine-containing, optically active quinolone compound which can be used as an anti-infection medicine. The compound has defined structure and stable properties. Compared with ulifoxacin, the compound of the invention has improved water solubility. It is readily soluble in water. It is of relatively strong antibacterial activity, low toxicity to kidneys, and of no irritation to skin and muscle. Its NOAEL is 30mg/kg, which is 10 times higher than that of ulifoxacin. Its side effects are low, which increases the clinical drug safety. It has a broad antimicrobial spectrum. Its antimicrobial activity is 1-3 times higher than that of the racemic ulifoxacin. In addition, the production process of the compound is simple, reasonable, and thus it has industrial applicability.

## Claims

1. An antibacterial drug, being **characterized** as a fluorine-containing, optically active quinolone compound 1: 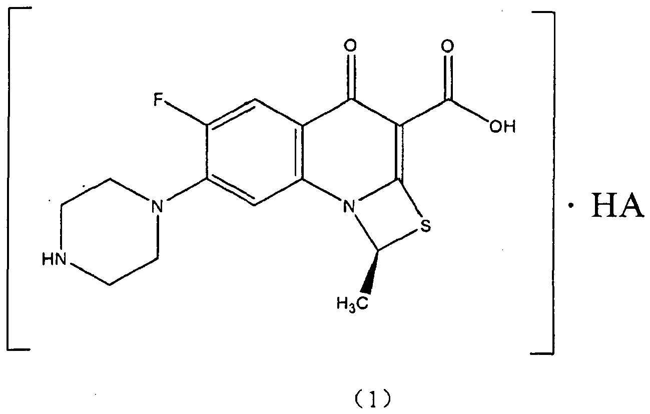 wherein HA is an organic acid selected from the group consisting of methylsulfonic acid, lactic acid, glutamic acid, gluconic acid and aspartic acid, which forms a pharmaceutically acceptable compound with (S)-6-fluoro-1-methyl-4-oxo-7-(1-piperazinyl)-1H,4H-[1,3]thiazeto[3,2-a]quinoline-3-carboxylic acid.

2. The antibacterial drug of claim 1, wherein the NA is lactic acid and compound 1 has the specific rotation [α]²⁰_{D} from -112.5 to -118.2° and IR absorptions at 1698 cm⁻¹, 1629 cm⁻¹, 1605 cm⁻¹, 1501 cm⁻¹, 1396 cm⁻¹, and 1257 cm⁻¹.

3. The antibacterial drug of claim 1, wherein the HA is methylsulfonic acid, and compound 1 has the specific rotation [α]²⁰_{D} from -106.6 to -112.6° and IR absorptions at 1707 cm⁻¹, 1629 cm⁻¹, 1602 cm⁻¹, and 1501 cm⁻¹.

4. The antibacterial drug of claim 1, wherein said organic acid is gluconic acid, and the product with (S)-6-fluoro-1-methyl-4-oxo-7-(1-piperazinyl)-1H,4*H-*[1,3]thiazeto[3,2-a]quinoline-3-carboxylic acid is (S)-6-fluoro-1-methyl-4-oxo-7-(1-piperazinyl)-1H,4*H*-[1,3]thiazeto[3,2-a] quinoline-3-carboxylic acid gluconate having IR absorptions at 1695 cm⁻¹, 1629 cm⁻¹, 1601 cm⁻¹, and 1503 cm⁻¹.

5. The antibacterial drug of claim 1, wherein said organic acid is glutamic acid, and the product with (S)-6-fluoro-1-methyl-4-oxo-7-(1-piperazinyl)-1H,4*H-*[1,3]thiazeto[3,2-a]quinoline-3-carboxylic acid is (S)-6-fluoro-1-methyl-4-oxo-7-(1-piperazinyl)-1H,4H-[1,3]thiazeto[3,2-a]quinoline-3-carboxylic acid glutamate having IR absorptions at 1628 cm⁻¹, 1603 cm⁻¹, 1499 cm⁻¹, 1457 cm⁻¹, 1397 cm⁻¹, and 1257 cm⁻¹.

6. The antibacterial drug of claim 1, wherein said organic acid is aspartic acid, and the product with (S)-6-fluoro-1-methyl-4-oxo-7-(1-piperazinyl)-1H,4*H-*[1,3]thiazeto[3,2-a]quinoline-3-carboxylic acid is (S)-6-fluoro-1-methyl-4-oxo-7-(1-piperazinyl)-1H,4H-[1,3]thiazeto[3,2-a]quinoline-3-carboxylic acid aspartate having IR absorptions at 1695 cm⁻¹, 1628 cm⁻¹, 1602 cm⁻¹, and 1499 cm⁻¹.

## Patentansprüche

1. Antibakterieller Wirkstoff, gekennzeichnet als fluorhaltige, optisch aktive Chinolonverbindung 1: 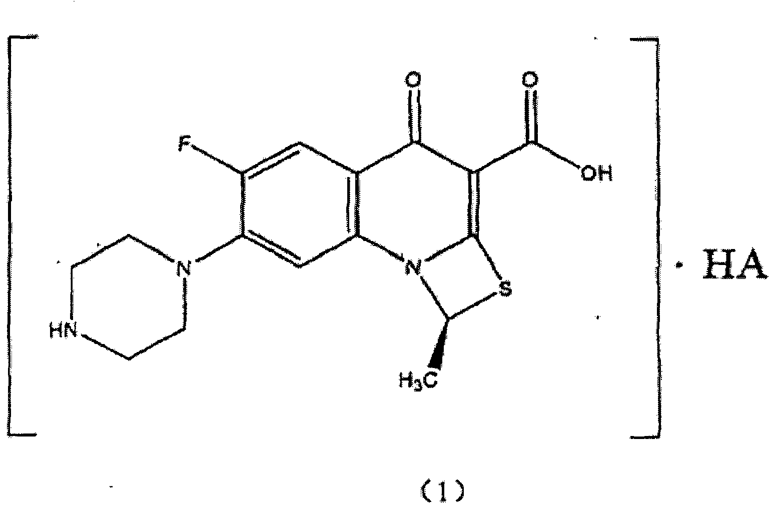 wobei HA eine organische Säure ist, die aus der Gruppe, die aus Methylsulfonsäure, Milchsäure, Glutaminsäure, Gluconsäure und Asparaginsäure besteht, ausgewählt ist und mit (S)-6-Fluor-1-methyl-4-oxo-7-(1-piperazinyl)-1H,4H-[1,3]thiazeto[3,2-a]chinolin-3-carbonsäure eine pharmazeutisch annehmbare Verbindung bildet.

2. Antibakterieller Wirkstoff gemäß Anspruch 1, wobei HA Milchsäure ist und Verbindung 1 einen spezifischen Drehwinkel [α]²⁰_{D} von -112,5° bis -118,2° und IR-Absorptionen bei 1698 cm⁻¹, 1629 cm⁻¹, 1605 cm⁻¹, 1501 cm⁻¹, 1396 cm⁻¹ und 1257 cm⁻¹ aufweist.

3. Antibakterieller Wirkstoff gemäß Anspruch 1, wobei HA Methylsulfonsäure ist und Verbindung 1 einen spezifischen Drehwinkel [α_{]}²⁰_{D} von -106,6° bis -112,6° und IR-Absorptionen bei 1707 cm⁻¹, 1629 cm⁻¹, 1602 cm⁻¹ und 1501 cm⁻¹ aufweist.

4. Antibakterieller Wirkstoff gemäß Anspruch 1, wobei es sich bei der organischen Säure um Gluconsäure und bei dem Produkt mit (S)-6-Fluor-1-methyl-4-oxo-7-(1-piperazinyl)-1H,4H-[1,3]thiazeto[3,2-a]chinolin-3-carbonsäure um (S)-6-Fluor-1-methyl-4-oxo-7-(1-piperazinyl)-1H,4H-[1,3]thiazeto[3,2-a]chinolin-3-carbonsäuregluconat mit IR-Absorptionen bei 1695 cm⁻¹, 1629 cm⁻¹, 1601 cm⁻¹ und 1503 cm⁻¹ handelt.

5. Antibakterieller Wirkstoff gemäß Anspruch 1, wobei es sich bei der organischen Säure um Glutaminsäure und bei dem Produkt mit (S)-6-Fluor-1-methyl-4-oxo-7-(1-piperazinyl)-1H,4H-[1,3]thiazeto[3,2-a]chinolin-3-carbonsäure um (S)-6-Fluor-1-methyl-4-oxo-7-(1-piperazinyl)-1H,4H-[1,3]thiazeto[3,2-a]chinolin-3-carbonsäureglutamat mit IR-Absorptionen bei 1628 cm⁻¹, 1603 cm⁻¹, 1499 cm⁻¹, 1457 cm⁻¹, 1397 cm⁻¹ und 1257 cm⁻¹ handelt.

6. Antibakterieller Wirkstoff gemäß Anspruch 1, wobei es sich bei der organischen Säure um Asparaginsäure und bei dem Produkt mit (S)-6-Fluor-1-methyl-4-oxo-7-(1-piperazinyl)-1H,4H-[1,3]thiazeto[3,2-a]chinolin-3-carbonsäure um (S)-6-Fluor-1-methyl-4-oxo-7-(1-piperazinyl)-1H,4H-[1,3]thiazeto[3,2-a]chinolin-3-carbonsäureaspartat mit IR-Absorptionen bei 1695 cm⁻¹, 1628 cm⁻¹, 1602 cm⁻¹ und 1499 cm⁻¹ handelt.

## Revendications

1. Agent antibactérien, caractérisé comme un composé quinolone fluoré, optiquement actif 1: 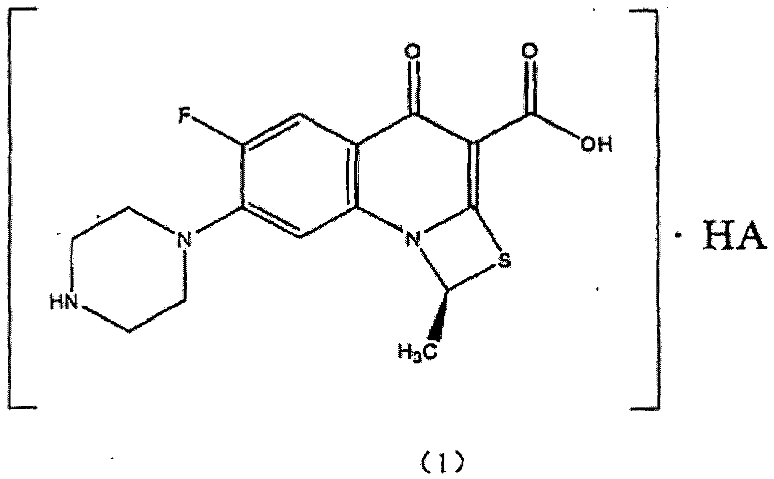 dans lequel HA est un acide organique choisi dans le groupe consistant en l'acide méthylsulfonique, l'acide lactique, l'acide glutamique, l'acide gluconique et l'acide aspartique, formant un composé pharmaceutiquement acceptable avec l'acide (S)-6-fluoro-1-méthyl-4-oxo-7-(1-pipérazinyl)-1H,4H-[1,3]thiazéto[3,2-a]quinoline-3-carboxylique.

2. Agent antibactérien selon la revendication 1, dans lequel le HA est l'acide lactique et le composé 1 a une rotation spécifique [α]²⁰_{D} de -112,5° à -118,2° et des absorptions infrarouge à 1698 cm⁻¹, 1629 cm⁻¹, 1605 cm⁻¹, 1501 cm⁻¹, 1396 cm⁻¹ et 1257 cm⁻¹.

3. Agent antibactérien selon la revendication 1, dans lequel le HA est l'acide méthylsulfonique et le composé 1 a une rotation spécifique [α]²⁰_{D} de -106,6° à -112,6° et des absorptions infrarouge à 1707 cm⁻¹, 1629 cm⁻¹, 1602 cm⁻¹ et 1501 cm⁻¹.

4. Agent antibactérien selon la revendication 1, dans lequel l'acide organique est l'acide gluconique et le produit avec l'acide (S)-6-fluoro-1-méthyl-4-oxo-7-(1-pipérazinyl)-1H,4H-[1,3]thiazéto[3,2-a]quinoline-3-carboxylique est le gluconate de l'acide (S)-6-fluoro-1-méthyl-4-oxo-7-(1-pipérazinyl)-1H,4H-[1,3]thiazéto[3,2-a]quinoline-3-carboxylique ayant des absorptions infrarouge à 1695 cm⁻¹, 1629 cm⁻¹, 1601 cm⁻¹ et 1503 cm⁻¹.

5. Agent antibactérien selon la revendication 1, dans lequel l'acide organique est l'acide glutamique et le produit avec l'acide (S)-6-fluoro-1-méthyl-4-oxo-7-(1-pipérazinyl)-1H,4H-[1,3]thiazéto[3,2-a]quinoline-3-carboxylique est le glutamate de l'acide (S)-6-fluoro-1-méthyl-4-oxo-7-(1-pipérazinyl)-1H,4H-[1,3]thiazéto[3,2-a]quinoline-3-carboxylique ayant des absorptions infrarouge à 1628 cm⁻¹, 1603 cm⁻¹, 1499 cm⁻¹, 1457 cm⁻¹, 1397 cm⁻¹ et 1257 cm⁻¹.

6. Agent antibactérien selon la revendication 1, dans lequel l'acide organique est l'acide aspartique et le produit avec l'acide (S)-6-fluoro-1-méthyl-4-oxo-7-(1-pipérazinyl)-1H,4H-[1,3]thiazéto[3,2-à]qùinoline-3-carboxylique est l'aspartate de l'acide (S)-6-fluoro-1-méthyl-4-oxo-7-(1-pipérazinyl)-1H,4H-[1,3]thiazéto[3,2-a]quinoline-3-carboxylique ayant des absorptions infrarouge à 1695 cm⁻¹, 1628 cm⁻¹, 1602 cm⁻¹ et 1499 cm⁻¹.
